# EUROPEAN PATENT APPLICATION

(11) **EP 4 782 832 A1**
(43) Date of publication of application: **29.07.2026**
(21) Application number: 26150023.5
(22) Date of filing: 02.01.2026
(51) Int. Cl.: G01N 33/36, G01N 33/34

(54) **TRUE CROSS-DIRECTIONAL WEB SCANNING APPARATUS**

(30) Priority: 22.01.2025 US 202519033781
(71) Applicant: Honeywell International Inc., Charlotte, NC 28202 (US)
(72) Inventor: THALORE, Sudhir, Charlotte, 28202 (US); REYNEN, Greg, Charlotte, 28202 (US); NAFISSI, Pezhman, Charlotte, 28202 (US)
(74) Representative: Haseltine Lake Kempner LLP

(57) **Abstract**

The apparatus and method guides a web (120) of material to move in a first direction and a second direction. Sensor signals are transmitted from a source module (210) slidably attached to a first rail (220b) and located at an angle to and between the web (120) of material moving in the first direction and the second direction. First and second receiver modules (212, 214) are each slidably attached to second and third rails (220a, 220c) positioned in alignment with the first rail (220b). The sensor module (210) cooperates with the first receiver module (212) to slidably travel along the first and second rails to capture measurements associated with the web (120) of material in the first direction and the sensor module (210) cooperates with the second receiver module (214) to slidably travel along the first and third rails to capture measurements associated with the web (120) of material in the second direction.

## Description

### TECHNICAL FIELD

This disclosure relates generally to scanning measurement systems. More specifically, this disclosure relates to scanning apparatus for a web of material having a scanning sensor assembly arranged in a stacked manner and fixed at an angle to the web of material.

### BACKGROUND

Sheets or other webs of material are used in a variety of industries and in a variety of ways. These materials can include paper, multi-layer paperboard, and other products manufactured or processed in long webs. As a particular example, long sheets of paper can be manufactured and collected in reels.

It is often necessary or desirable to measure one or more properties of a web of material as the web is being manufactured or processed. Adjustments can then be made to the manufacturing or processing system to ensure that the properties stay within desired ranges. Measurements are often taken using one or more scanning heads that move back and forth across the width of the web.

### SUMMARY

This disclosure relates to a scanning sensor apparatus and method arranged in a stacked manner and fixed at an angle to a web of material.

An apparatus is disclosed that comprises a web of material guided to move in a first direction and arranged to be directed to move in a second direction. A source module disposed to transmit sensor signals is slidably attached to a first rail. The first rail is located at an angle to and between the web of material in the first direction and the second direction. A first receiver module for receiving the sensor signals from the source module is slidably attached to a second rail. The second rail is positioned in alignment with the first rail and located on a first side of the web of material in the first direction. A second receiver module for receiving the sensor signals from the source module is slidably attached to a third rail. The third rail is positioned in alignment with the first rail and located on a first side of the web of material in the second direction. The sensor module cooperates with the first receiver modules to slidably travel along the first and second rails to capture measurements associated with the web of material in the first direction and to cooperate with the second receiver module to slidably travel along the first and third rails to capture measurements associated with the web of material traveling in the second direction.

A method is also disclosed comprising guiding a web of material to move in a first direction and also in a second direction. Sensor signals are transmitted from at least one sensor slidably attached to a first rail. The first rail is located at an angle to and between the web of material moving in the first direction and the second direction. The method further includes slidably attaching a first receiver to a second rail positioned in alignment with the first rail and located on a first side of the web of material in the first direction. The first receiver arranged to receive the sensor signals from the at least one sensor and slidably attaching a second receiver to a third rail positioned in alignment with the first rail and located on a first side of the web of material in the second direction. The second receiver arranged to receive the sensor signals from the at least one sensor, wherein the first receiver cooperates with the at least one sensor to slidably travel along the first and second rails to capture measurements associated with the web of material moving in the first direction and the second receiver cooperates with the at least one sensor to capture measurements associated with the web of material from the web of material in the second direction.

Other technical features may be readily apparent to one skilled in the art from the following figures, descriptions, and claims.

### BRIEF DESCRIPTION OF THE DRAWINGS

For a more complete understanding of this disclosure, reference is now made to the following description, taken in conjunction with the accompanying drawings, in which:
FIG. 1 is a schematic illustration of an exemplary true cross-directional measurement system in accordance with the present disclosure.
FIG. 2 is a diagram of a true-CD measurement profile developed by the true cross-directional measurement system of FIG. 1 in accordance with the present disclosure.

### DETAILED DESCRIPTION

The figures discussed below, and the various embodiments used to describe the principles of the present invention in this patent document are by way of illustration only and should not be construed in any way to limit the scope of the invention. Those skilled in the art will understand that the principles of the invention may be implemented in any type of suitably arranged device or system.

Scanning systems for web-manufacturing or other web-related processes often use translating scanning heads that house sensors and that move back and forth across each side of the web. The web can represent any suitable material or materials manufactured or processed as moving sheets or other webs. For example, a web can include paper, multi-layer paperboard, cardboard, plastic, films, textiles, or metal webs. In many systems, scanning heads are mechanically coupled to a belt system mounted to a frame and that is driven by a single or multiple motors. The web is transported through a portion of the scanning system using two pairs of rollers. For example, a roller pair can pull the web from a previous stage of a web-manufacturing or web-processing system and feed the web into a subsequent stage of the web-manufacturing or web-processing system. The roller pairs move the web in a direction referred to as the machine direction (MD).

The scanning system is comprised of one or more scanning sensor assemblies positioned between the rollers. Each scanning sensor assembly may include one or more sensors capable of measuring at least one characteristic of the web. For example, the scanning sensor assemblies could include sensors for measuring the moisture, caliper, anisotropy, basis weight, color, gloss, sheen, haze, surface features (such as roughness, topography, or orientation distributions of surface features), or any other or additional characteristic(s) of the web. In general, a characteristic of the web can vary along the length of the web in the machine direction and/or across the width of the web in a cross-direction (CD). Typically, in such scanning systems the cross-direction scanning is made across the web at the same time as the web is transported in the machine direction providing an angular scanning profile from one edge to an opposite edge of the web that is dependent on the speed by which the web is transported in the machine direction and the speed of displacement of the scanning head in the cross-direction from the one edge of the web to the other.

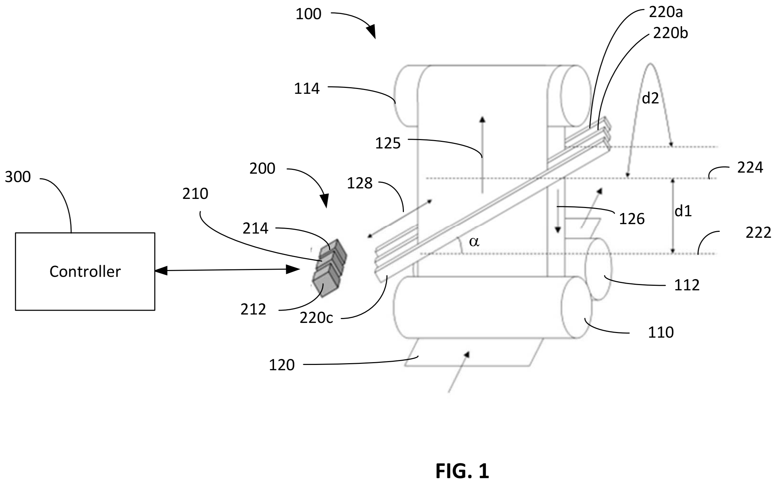

This invention proposes a new method for cross-direction scanning involving scanning sensor assemblies arranged in a stacked manner that scan at a fixed angle to the web of material, thereby, allowing the creation of true cross-directional (true-CD) scanning patterns when the velocity of the web is matched by the machine-direction component of the scanning head velocity. This allows creation of cross-direction profile measurements independent of machine direction variations and machine direction profile measurements that are less affected by cross-direction variations compared to traditional scanning systems. The true-CD scanning system can also be used for patch coating measurement applications that can provide scan profiles that avoid coating gaps. Since machine direction space is at a premium in web-manufacturing systems, the true-CD scanning system of the present disclosure may be implemented using a vertically oriented scanning frame with the web of material passing through gaps in rails attached to the frame at a pass-angle of 90 degrees to the stacked sensor assembly, as shown in FIG. 1

FIG. 1 illustrates an exemplary embodiment of the true-CD scanning system 100 of the present disclosure. The scanning system 100 comprises three feed rollers 110, 112 and 114 attached to a frame (the frame is not shown for clarity) that move a web of material 120 through a scanning assembly 200. The scanning assembly 200 is fixed at a tilt angle α of approximately 45 degrees to the MD illustrated by arrows 125. The scanning assembly 200 includes a stacked scanning system arrangement comprising a source module 210 and two receiver modules 212, 214. The receiver modules 212, 214 are located on either side of source module 210. A first scanning head is formed by the source module 210 and receiver module 212 and a second scanning head is formed by source module 210 and receiver module 214.

Source module 210 includes any suitable structure for carrying one or more web sensors arranged to transmit sensor signals into the material conveyed by the web 120 and to be received by receiver modules 212 and 214. The receiver modules 212, 214 receive the sensor signals that pass through the materials to capture measurements associated with the web 120. Each web sensor may include any suitable structure for transmitting specific measurements associated with one or more characteristics of web 120. Each receiver module 212, 214 may include any suitable structure for receiving the sensor signals sent by the source module 210. A web sensor could represent a contact sensor that takes measurements of a web via contact with the web 120 or a noncontact sensor that takes measurements of the web 120 without contacting the web.

Power can be provided to source module 210 and receiver modules 212, 214 in any suitable manner. For example, source module 210 and receiver modules 212, 214 may be coupled to one or more cables that provide power to modules 210, 212 and 214. The scanning assembly 200 could further include an internal power supply, such as a battery or an inductive coil used to receive power wirelessly.

Each receiver module 212, 214 of scanning assembly 200 can send sensor measurement data to an external controller 300. The controller 300 could use the measurement data in any suitable manner. For example, the controller could use the measurement data to generate CD profiles of the web 120. The controller could then use the CD profiles to determine how to adjust the properties of the web 120. The controller could also use the CD profiles or measurement data to support monitoring applications, process historian applications, or other process control-related applications.

The source module 210 and the first and second receiver modules 212, 214 are each slidably attached to a respective one of rails 220a, 220b and 220c that are fixed angularly across the web 120. The source module 210 and each first and second receiver modules 212, 214 may be attached to a carriage (not shown). Each carriage can traverse back and forth along rails 220a-220c to move the source module 210 and the first and second receiver modules independently across the web 120 in direction 128. Each rail 220a-220c generally includes any suitable structure on which the carriage and the attached modules 210, 212, 214 can move, such as a belt, shaft, or beam formed of metal or another suitable material. Each carriage includes any suitable structure for moving along rails 220a-220c. Each module 210, 212, 214 is arranged to be driven by any appropriate means to travel along rails 220-220c to scan back and forth over the web 120, individually or as scanning head pairs. For example, the source module may be arranged to travel as stacked pair with the first receiver module as a first scanning head or travel as a stacked pair with the second receiver module as a second scanning head. Additionally, the source module 210 by also arranged to travel in a stacked assembly comprising the source module and both the receiver modules 212, 214. The web 120 in the MD 125 transverses between the source module 210 and the receiver module 212 of the first head and the source module 210 and receiver module 214 of the second head.

In another embodiment, the scanning assembly 200 may contain only one pair of scanning rails. For example, source module 210 and the first receiver module 212 may each be slidably attached to a respective one of rails 220b and 220c to a carriage (not shown) to traverse back and forth along rails 220b-220c to move as a first scanning head across the web 120 in direction 128. The first scanning head moves along the web 120 during the forward scan 125 only to produce a true-CD profile. In still another embodiment multi-axis motion systems (not shown) may be used to move the source 210 and receiver modules 212, 214 along the true-CD scanning pattern described herein. For the sake of simplicity, however, the vertically oriented setup shown in FIG. 1 will be used to explain the present disclosure. Also, the operation of the scanning assembly 200 will be explained using the source module 210 and receiver module 212 comprising the first scanning head and the source module 210 and receiver module 214 comprising the second scanning head.

The web 120 passes between the source and receiver modules of the first and second scanning heads at a vertical pass line angle. The distance "d1" is the sheet distance between the low-end 222 and the high-end 224 scanning points established by rails 220a-220c. The distance "d2" is the sheet distance between the first scanning head and the second scanning head when they are at a high-end scanning point 224.

In the embodiment shown in FIG.1 , each first and second scanning head can be made to follow an angular scan path 128 across the web 120, when the scanning speed, e.g. the speed of the scanning heads transversing the rails 220a-220c, is set to (1/sin(α)) times the speed of the web 120 in the MD direction 125. For the simplest case, a tilt angle α set at 45° scanning speed is √2 times the speed of the web, at a constant rate of speed while over the web 120. The first scanning head will be moving along the web 120 during the forward scan, producing a true-CD profile. The second scanning head will be doing the same in a reverse scan, when the web 120 is moving in the direction as illustrated by arrow 126.

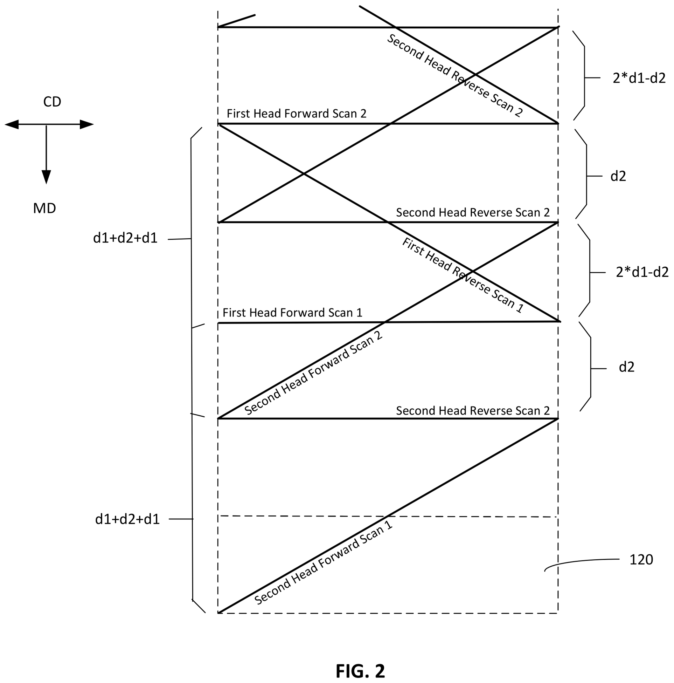

FIG. 2 illustrates the scan paths of the first scanning and second scanning heads across the web 120 for the embodiment of FIG. 1. When both the first and second scanning heads are at the low-end scan point 222, they are separated by a sheet distance of d1 + d2 + d1. During the forward scan in direction 125, the first scanning head moves along with web 120 in direction 128 across the web 120 following a true-CD path. The second head moves in a crisscross path as in traditional scanners. At the end of the forward scan, both the first and the second heads are at the high-end scan point 224 and separated by distance d2. During the reverse scan of web 120 in direction 126, the second head moves along with the MD following the true-CD path. The first scanning head moves in the traditional crisscross path. When both the first and second heads reach the first low end scan point 222, the sheet distance between the heads will again be at a sheet distance of d1 + d2 + d1.

As shown in diagram of FIG. 2, a true-CD measurement profile is created every (2*d1-d2) and d2 intervals by each of the first and second scanning heads. By adjusting the sheet path such that distance d2 is equal to distance d1, evenly spaced true-CD measurements can be produced. It can be seen that evenly spaced true-CD measurements can also be produced when distance d2 is an odd multiple of distance d1 as described in Table 1.

Table 1 below lists scans for various cases where distance d2 is various odd multiples of distance d1. Note that the true-CD scans may need to be re-ordered based on the MD position when the d1-to-d2 multiple is greater than 1. The MD position measurement assigned to controller 300 can be used to re-order the scans for this purpose. Also note that when d1-to-d2 multiple is greater than 1, contiguous true-CD scans start after (dl-to-d2 multiple - 1)* d1 distance from the first true-CD scan.

**Table 1**

| True-CD Scan Number | Distance from Previous True-CD Scan | Distance of Each True-CD Scan from True-CD Scan 1 | | | | |
|---|---|---|---|---|---|---|
| | | General Case (d1 is not equal to d2) | d2 = d1 | d2 = 3d1 | d2 = 5d1 | d2 = 7d1 |
| | | | Substitute d2 in Column 3 with multiple of d1 above | | | |
| 2 | d2 | d2 | d1 | 3d1 | 5d1 | 7d1 |
| 3 | 2*d1 - d2 | 2d1 | 2d1 | 2d1 | 2d1 | d1 |
| 4 | d2 | 2d1 + d2 | 3d1 | 5d1 | 7d1 | 9d1 |
| 5 | 2*d1 - d2 | 4d1 | 4d1 | 4d1 | 4d1 | 4d1 |
| 6 | d2 | 4d1 + d2 | 5d1 | 7d1 | 9d1 | 11d1 |
| 7 | 2*d1 - d2 | 6d1 | 6d1 | 6d1 | 6d1 | 6d1 |
| 8 | d2 | 6d1 + d2 | 7d1 | 9d1 | 11d1 | 13d1 |
| 9 | 2*d1 - d2 | 8d1 | 8d1 | 8d1 | 8d1 | 8d1 |
| 10 | d2 | 8d1 + d2 | 9d1 | 11d1 | 13d1 | 15d1 |
| 11 | 2*d1 - d2 | 10d1 | 10d1 | 10d1 | 10d1 | 10d1 |
| 12 | d2 | 10d1 + d2 | 11d1 | 13d1 | 15d1 | 17d1 |
| 13 | 2*d1 - d2 | 12d1 | 12d1 | 12d1 | 12d1 | 12d1 |
| 14 | d2 | 12d1 + d2 | 13d1 | 15d1 | 17d1 | 19d1 |
| 15 | 2d1 - d2 | 14d1 | 14d1 | 14d1 | 14d1 | 14d1 |
| 16 | d2 | 14d1 + d2 | 15d1 | 17d1 | 19d1 | 21d1 |
| 17 | 2d1 - d2 | 16d1 | 16d1 | 16d1 | 16d1 | 16d1 |
| 18 | d2 | 16d1 + d2 | 17d1 | 19d1 | 21d1 | 23d1 |
| 19 | 2d1 - d2 | 18d1 | 18d1 | 18d1 | 18d1 | 18d1 |
| 20 | d2 | 18d1 + d2 | 19d1 | 21d1 | 23d1 | 25d1 |

In addition, traditional crisscross profiles may also be created by each head in between the true-CD profiles which can be used to generate true MD profiles by removing the mean CD profile from it. Since true-CD profile measurements are available, this process can result in much better MD profile measurement compared to traditional scanning systems.

Distance d1 may be decreased to provide more closely spaced CD profiles by decreasing the tilt angle α, to approximately 30 degrees. However, the speed by which the first and second scanning heads traverse the rails (head speed) must be increased to twice the speed of the web 120 in the MD (1/sin(30)) to achieve the same functionality. Another advantage of a lesser tilt angle as compared to the 45 degree tilt angle is that the frame would require less vertical height and a shorter frame length, so that the cost of the frame will be lower.

Conversely, the head speed can be reduced by increasing the tilt angle α. A tilt angle of approximately 60 degrees requires 1.1547 times (1/sin(60)) the sheet speed. However, this setup will require more vertical space and more frame material, increasing the overall cost of the scanning system.

The tilt angle of the scanning assembly 200 may be used to provide linear product coverage. Linear product coverage is the total length of scan per unit distance of the product being manufactured by the web manufacturing process. Table 2 compares linear product coverage for two tilt angles of 45 degrees and 30 degrees to traditional scanning heads that scan orthogonal to the moving sheet at the same scan speed. The comparison is made for the total length of frame material required in each case. The results shown in Table 2 makes use of the fact that the vertical setup described above with a tilt angle α° is equivalent to two horizontal frames angled at (90 - α)° to the MD of web 120.

**Table 2**

| | **Traditional Scan Heads** | **Tilt Angle 45°** |
|---|---|---|
| **Scan Speed** | √2 times Sheet Speed | √2 times Sheet Speed |
| **Linear Product Coverage** | 3.46 | 3.24 |
| **Frame Material Needed** | 4 times Sheet Width | 4.24 times Sheet Width |

| | **Traditional Scan Heads** | **Tilt Angle 30°** |
|---|---|---|
| **Scan Speed** | 2 times Sheet Speed | 2 times Sheet Speed |
| **Linear Product Coverage** | 4.47 | 4.38 |
| **Frame Material Needed** | 4 times Sheet Width | 3.46 times Sheet Width |

One application of the true-CD scanning system of Fig. 1 is in sheet manufacturing systems that involve measurement of patch coatings, such as for example, intermittent, pattern, or discontinuous patches used in the manufacture of lithium-ion battery anode and cathodes. When traditional scanners are used to measure a patch coating processes, the gap between patches in coating will appear at different positions in each scan. This makes it difficult to build the entire coat profile and to use the measurement in control applications. The true-CD scanning method described above can be used to eliminate the coating gaps in scanning of patch coatings. Traditional scanners may be arranged to scan diagonally across a patch, but this requires a much larger patch. The true-CD scanning method of the present invention allow coating gaps to be avoided, even with narrow patches. The true-CD of scanning assembly 200 can be arranged to scan over alternate patches or, if the patches are very narrow, over different patches. The return scan after each true-CD scan ensures that some part of all the patches will be scanned.

The true-CD scanning method also provides benefits to the problem of MD variation aliasing. MD variations can be aliased into CD profile measurements when the frequency of the MD variation matches the scan frequency. In these cases, it is impossible to properly disentangle the MD variation from the CD variation. A true-CD profile is inherently immune to MD aliasing due to the true-CD profiles providing the full magnitude of a stepwise change in the first scan.

It may be advantageous to set forth definitions of certain words and phrases used throughout this patent document. The term "communicate," as well as derivatives thereof, encompasses both direct and indirect communication. The terms "include" and "comprise," as well as derivatives thereof, mean inclusion without limitation. The term "or" is inclusive, meaning and/or. The phrase "associated with," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, have a relationship to or with, or the like. The phrase "at least one of," when used with a list of items, means that different combinations of one or more of the listed items may be used, and only one item in the list may be needed. For example, "at least one of: A, B, and C" includes any of the following combinations: A, B, C, A and B, A and C, B and C, and A and B and C.

The description in the present application should not be read as implying that any particular element, step, or function is an essential or critical element that must be included in the claim scope. The scope of patented subject matter is defined only by the allowed claims. Moreover, none of the claims is intended to invoke 35 U.S.C. § 112(f) with respect to any of the appended claims or claim elements unless the exact words "means for" or "step for" are explicitly used in the particular claim, followed by a participle phrase identifying a function. Use of terms such as (but not limited to) "mechanism," "module," "device," "unit," "component," "element," "member," "apparatus," "machine," "system," or "controller" within a claim is understood and intended to refer to structures known to those skilled in the relevant art, as further modified or enhanced by the features of the claims themselves and is not intended to invoke 35 U.S.C. § 112(f).

While this disclosure has described certain embodiments and generally associated methods, alterations and permutations of these embodiments and methods will be apparent to those skilled in the art. Accordingly, the above description of example embodiments does not define or constrain this disclosure. Other changes, substitutions, and alterations are also possible without departing from the spirit and scope of this disclosure, as defined by the following claims.

## Claims

1. An apparatus comprising:
a web of material guided to move in a first direction;
a source module disposed to transmit sensor signals slidably attached to a first rail the first rail located at an angle to and between the web of material in the first direction;
a first receiver module for receiving the sensor signals from the source module, the first receiver module slidably attached to a second rail, the second rail positioned in alignment with the first rail and located on a first side of the web of material in the first direction,
wherein the source module cooperates with the first receiver module to slidably travel along the first and second rails to capture measurements associated with the web of material in the first direction.

2. The apparatus of claim 1, wherein the web of material is arranged to move in a second direction and the apparatus further includes:
a second receiver module for receiving the sensor signals from the source module, the second receiver module slidably attached to a third rail, the third rail positioned in alignment with the first rail and located on a first side of the web of material in the second direction,
wherein the source module cooperates with the second receiver module to slidably travel along the first and third rails to capture measurements associated with the web of material traveling in the second direction.

3. The apparatus of claim 2, wherein the web of material is guided by at least a first and a second roller, the first roller guiding the web of material to move in the first direction and the second roller guiding the web of material to move in the second direction, wherein the second direction is opposite to the first direction.

4. The apparatus of claim 3, wherein the first rail is located in a cross direction to the web of material first and second direction between the first and second rollers and the first rail includes a low-end scanning point by the first roller and a high-end scanning point located by the second roller.

5. The apparatus of claim 4, wherein the second rail is in a stacked alignment in a cross direction to the web of material wherein the web of material in the first direction passes between the first rail and the second rail.

6. The apparatus of claim 5, wherein the third rail is in a stacked alignment in a cross direction to the web of material wherein the web of material in the second direction passes between the first rail and the third rail.

7. The apparatus of claim 6, wherein the source module and the first receiver module travel in a cross direction as a first scanning head that cooperate to capture measurements associated with the web of material from the first side of the web of material moving in the first direction and wherein the source module and the second receiver module travel in a cross direction as a second scanning head that cooperate to capture measurements associated with the web of material from the first side of the web of material moving in the second direction.

8. The apparatus of claim 2, wherein the first, the second and the third rails are located at an angle of 90 degrees or less to the web of material first and second direction.

9. A method comprising:
guiding a web of material a web of material to move in a first direction;
transmitting sensor signals from at least one sensor slidably attached to a first rail, the first rail located at an angle to and between the web of material moving in the first direction;
slidably attaching a first receiver to a second rail, the second rail positioned in alignment with the first rail and located on a first side of the web of material in the first direction, the first receiver arranged to receive the sensor signals from the at least one sensor,
wherein the first receiver cooperates with the at least one sensor to slidably travel along the first and second rails to capture measurements associated with the web of material in the first direction.

10. The method of claim 9, wherein the web of material is directed in a second direction and the method further includes:
slidably attaching a second receiver to a third rail, the third rail positioned in alignment with the first rail and located on a first side of the web of material in the second direction, the second receiver arranged to receive the sensor signals from the at least one sensor,
wherein the second receiver cooperates with the at least one sensor to capture measurements associated with the web of material from the web of material in the second direction.

11. The method of claim 10, wherein the web of material is guided to move in the first direction by a first roller and to move in the second direction by a second roller the second direction opposite the first direction.

12. The method of claim 11, wherein the first rail is angularly located between the web of material in the first and second direction and between the first and second rollers from a low-end scanning point located near the first roller to a high-end scanning point located by the second roller.

13. The method of claim 11, wherein the second rail is in a stacked alignment with the first rail, wherein the web of material in the first direction passes between the first rail and the second rail and the third rail is in a stacked alignment with the first rail, wherein the web of material in the second direction passes between the first rail and the third rail.

14. The method of claim 13, wherein the at least one sensor and the first receiver form a first scanning head that cooperate to capture measurements associated with the web of material from a first side of the web of material moving in the first direction and the at least one sensor and the second receiver form a second scanning head that cooperate to capture measurements associated with the web of material from a first side of the web of material moving in the second direction.

15. The method of claim 14, wherein a controller receives the measurements captured by the first and second receivers to generate cross direction profiles of the web of materials and uses the cross-direction profiles to support monitoring, process historian or process control applications.
